(19) Europäisches Patentamt
European Patent Office
Office européen des brevets

(11) Veröffentlichungsnummer : **0 130 582**
**B1**

(12) # EUROPÄISCHE PATENTSCHRIFT

(45) Veröffentlichungstag der Patentschrift : **06.05.87**

(51) Int. Cl.[4] : **C 07 C 53/08**, C 07 C 69/14, C 07 C 51/12, C 07 C 67/36

(21) Anmeldenummer : **84107503.9**

(22) Anmeldetag : **28.06.84**

(54) **Verfahren zur Herstellung von Essigsäure und Methylacetat.**

(30) Priorität : **01.07.83 DE 3323654**

(43) Veröffentlichungstag der Anmeldung : **09.01.85 Patentblatt 85/02**

(45) Bekanntmachung des Hinweises auf die Patenterteilung : **06.05.87 Patentblatt 87/19**

(84) Benannte Vertragsstaaten : **BE DE FR GB IT NL**

(56) Entgegenhaltungen :
**EP-A- 0 048 174**
**DE-B- 1 966 695**
**DE-B- 2 303 271**

(73) Patentinhaber : **BASF Aktiengesellschaft**
**Carl-Bosch-Strasse 38**
**D-6700 Ludwigshafen (DE)**

(72) Erfinder : **Mueller, Franz-Josef, Dr.**
**Mueller-Thurgau-Weg 1**
**D-6706 Wachenheim (DE)**
Erfinder : **Matt, Dominique, Dr.**
**16, Rue de Dunkerque**
**F-67000 Strasbourg (FR)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 130 582 B1

**Beschreibung**

Die vorliegende Erfindung betrifft ein verbessertes Verfahren zur Herstellung von Essigsäure und Methylacetat durch Carbonylierung von Methanol in der Gasphase in Gegenwart von Nickel enthaltenden Katalysatoren sowie in Gegenwart von Chlor, Brom oder Jod oder flüchtigen Verbindungen dieser Halogene als Promotoren :

$$CH_3—OH \xrightarrow[Hal]{CO\ ;\ Ni} CH_3—COOH + CH_3—COO—CH_3$$

Außerdem betrifft die Erfindung neue Katalysatoren, die zur Herstellung von Essigsäure und Methylacetat durch Gasphasencarbonylierung geeignet sind.

Die Herstellung von Essigsäure und Methylacetat durch Carbonylierung von Methanol in Gegenwart von carbonylbildenden Metallen und von Halogenen oder Halogenverbindungen ist allgemein bekannt.

Bei den großtechnisch ausgeübten Verfahren handelt es sich um Flüssigphasen-Verfahren mit Cobalt oder Rhodium als carbonylbildenden Metallen. Beide Verfahren vermögen jedoch nicht gänzlich zu befriedigen, weil die Verwendung von Cobalt einen hohen Druck (etwa 250-700 bar) und damit einen entsprechend großen Energieaufwand erfordert, und weil Rhodium, obwohl es einen geringeren Druck (etwa 35-70 bar) ermöglicht, extrem teuer ist.

Nach dem Verfahren der DE-A 1 005 949 nimmt man die Carbonylierung des Methanols in der Gasphase bei relativ geringem Druck an Aktivkohlekontakten vor, die mit Nickeljodid imprägniert sind. Problematisch ist hierbei jedoch, daß das Nickeltetracarbonyl, welches sich unter den Reaktionsbedingungen bildet, vom Trägermaterial desorbiert wird. Hierdurch nimmt die Aktivität des Katalysators allmählich ab, und gleichzeitig gibt das in den Produktaustrag gelangende Nickeltetracarbonyl zu Aufarbeitungsschwierigkeiten Anlaß. Diesen Nachteilen wird nach der Lehre der DE-A cit. im Prinzip dadurch abgeholfen, daß man das desorbierte Nickeltetracarbonyl an einem im Reaktionsraum nachgeschalteten katalysatorfreien Kohlekontakt wieder adsorbiert und die Strömungsrichtung der Einsatzstoffe umkehrt, wenn der erste Kontakt weitgehend an Nickel verarmt ist, das Nickel sich im zweiten aber entsprechend angereichert hat. Wenn die Nickelkonzentration sich in den beiden Kontaktoren abermals umgekehrt hat, ist die Strömungsrichtung der Reaktanten ebenfalls wieder zu ändern und so fort.

Da diese Arbeitsweise verfahrenstechnisch evident beschwerlich und damit unwirtschaftlich ist, lag der vorliegenden Erfindung die Aufgabe zugrunde, die Gasphasencarbonylierung des Methanols in Gegenwart von Nickelkatalysatoren effizienter zu gestalten.

Demgemäß wurde ein verbessertes Verfahren zur Herstellung von Essigsäure und Methylacetat durch Gasphasencarbonylierung von Methanol in Gegenwart von Nickel enthaltenden Katalysatoren sowie in Gegenwart von Chlor, Brom oder Jod oder flüchtigen Verbindungen dieser Halogene als Promotoren gefunden, welches dadurch gekennzeichnet ist, daß man hierzu Katalysatoren verwendet, deren aktive Masse aus mindestens 40 Gew.% Nickel besteht und die pro Gew.-Teil Nickel 0,001-1 Gew.-Teile Palladium enthalten.

Es wurde weiterhin gefunden, daß es besonders zweckmäßig ist, die erfindungsgemäßen Katalysatoren in Form von Trägerkatalysatoren einzusetzen.

Die Erfindung beruht auf der Beobachtung, daß die unerwünschte Bildung des flüchtigen Nickeltetracarbonyls durch die Gegenwart des Palladiums weitgehend zurückgedrängt wird und daß die Katalysatoren deshalb eine praktisch unbegrenzte Lebenszeit haben. Vermutlich bilden sich unter den Reaktionsbedingungen partielle Ni- und Pd-Carbonylkomplexe, die an die metallische Phase gebunden bleiben, oder sogenannte Haufenkomplexe (« Cluster ») mit mehreren Zentralatomen, die infolge ihres hohen Molekulargewichtes nicht mehr flüchtig sind.

Wesentlich sind daher Nickel und Palladium als Bestandteile der aktiven Masse. Die Gegenwart weiterer, unter den Reaktionsbedingungen reduzierbarer Metalle, auch von solchen, die keine Carbonyle bilden, stört hingegen zumindest unter dem Aspekt der Flüchtigkeit der Carbonyle nicht.

Gut geeignete Katalysatoren sind beispielsweise solche, deren aktive Masse

40 -90 Gew.% Ni
0,02 -10 Gew.% Pd und
0 -rd. 60 Gew.% Cu

enthält.

In welcher Form die aktive Masse unter der Reaktionsbedingungen vorliegt, ist nicht bekannt, jedoch ist anzunehmen, daß sich Legierungen oder legierungsähnliche Agglomerate bilden.

Es ist möglich, die Katalysatoren als Substanzkatalysatoren einzusetzen, etwa in Legierungsform oder in Form von Preßlingen der Metallsalze in entsprechender Zusammensetzung, wobei die Salze unter den Reaktionsbedingungen zu den Metallen bzw. zu deren Legierungen reduziert und danach an der Oberfläche in die aktive Carbonylform überführt werden.

Technisch sinnvoller ist es jedoch, die Katalysatoren in Form von Trägerkatalysatoren anzuwenden, die ungefähr 5 bis 15 Gew.% der Metalle (als Metall gerechnet) als aktive Masse enthalten.

Als Trägermaterialien eignen sich im Prinzip alle Substanzen, die sich unter den Reaktionsbedingungen praktisch inert verhalten, also die auch für sonstige Katalysatoren üblichen Materialien wie $SiO_2$, $TiO_2$, MgO und $Al_2O_3$, besonders jedoch Aktivkohle, wobei Stoffe mit großer innerer Oberfläche und damit entsprechend großem Adsorptionsvermögen bevorzugt werden.

Die Trägerteilchen können beliebige Gestalt (z. B. Kugeln, Zylinder, Stränge und Ringe) haben, sollten aber vorzugsweise zumindest in einer Dimension eine größere Ausdehnung als 2 mm aufweisen und andererseits nur in einer Dimension mehr als 10 mm messen.

Die Herstellung der Trägerkatalysatoren kann nach den hierfür gebräuchlichen Methoden erfolgen, etwa indem man das Trägermaterial mit Lösungen von Verbindungen der Metalle imprägniert, das Material trocknet und diese Vorgänge gegebenenfalls so oft wiederholt, bis der gewünschte Metallgehalt erreicht ist.

Als Lösungen der Metallverbindungen kommen insbesondere wäßrige, alkoholische oder wäßrigalkoholische Lösungen der Chloride oder vor allem der Nitrate in Betracht. Man kann diese Lösungen auch auf die Trägermaterialien aufsprühen.

Unter den Reaktionsbedingungen werden die Metallverbindungen dann zu den Metallen reduziert, jedoch empfiehlt es sich, die Reduktion vorher gesondert vorzunehmen, etwa indem man die Katalysatoren bei 150-450 °C und 1-100 bar 3-24 Stunden lang mit Wasserstoff behandelt. Es kann auch zweckmäßig sein, die reduktive Hydrierung nach jedem einzelnen Trockenvorgang vorzunehmen, damit bereits aufgebrachtes Material nicht wieder in Lösung geht.

Ferner ist es zweckmäßig, die imprägnierte Masse nach der Trocknung mit gasförmigem Ammoniak nachzubehandeln. Hierbei bilden sich vermutlich zum Teil Ni-$NH_3$-Komplexe, die sich leichter reduzieren lassen und die bei der Reduktion ein feinteiligeres und daher aktiveres Nickel liefern.

Die Hydrierung kann als vorgeschaltete Maßnahme im gleichen Reaktor erfolgen wie die Carbonylierung.

Weiterhin ist es möglich, die Komponenten getrennt auf das Trägermaterial aufzubringen und einen bereits vorgefertigten Nickelkatalysator zum Schluß mit Palladium zu dotieren. Diese Methode gestattet es vor allem, mit den geringen Pd-Mengen des definitionsgemäßen Bereichs auszukommen. In diesem Falle ist es ferner zweckmäßig, das Palladium aus organischer Lösung auf den vorgefertigten Katalysator aufzubringen, z. B. aus Lösungen des Palladium-bis-(dibenzal)acetons in Toluol oder Tetrahydrofuran. Die Bereitung des Katalysators in umgekehrter Reihenfolge — erst Aufbringen des Palladiums, dann des Nickels und gegebenenfalls weiterer Komponenten — ist ebenfalls möglich.

Die Menge der Katalysatoren hängt, wie stets bei heterogenen Kontakten, weitgehend von ihrer Oberfläche ab und kann daher nur näherungsweise angegeben werden. In der Regel wird man es so einrichten, daß pro Liter Reaktionsraum 50-250 g aktive Masse zur Verfügung stehen.

Die Carbonylierungsbedingungen sind für das vorliegende Verfahren nicht erfindungskritisch und bedürfen daher prinzipiell keiner Limitierung. Andererseits kommen für den praktischen Betrieb nur solche Bedingungen in Betracht, bei denen die Reaktion hinreichend schnell verläuft, bei denen keine merklich störende Methanbildung stattfindet und die auch nicht mit unnötig hohem Energieverbrauch für die Erzeugung des Reaktionsdrucks verbunden sind. Diese praktisch bedingten Bereiche liegen hinsichtlich der Temperatur zwischen 200 und 350, vorzugsweise 280 und 320 °C und hinsichtlich des CO-Partialdrucks zwischen 0,5 und 10 bar, entsprechend einem Gesamtdruck von etwa 30 bar.

Als Promotoren dienen Chlor, Brom oder vor allem Jod oder flüchtige Verbindungen diese Halogene, z. B. HCl, HBr oder HJ oder insbesondere organische Halogenverbindungen. Auf den organischen Rest dieser Verbindungen kommt es prinzipiell nicht an, zumal sich unter den Reaktionsbedingungen ohnehin die Methylhalogenide als stabilste Verbindungen bilden. Im Hinblick auf die Aufarbeitung der Reaktionsgemische ist es daher am zweckmäßigsten, von vornherein die Methylhalogenide, darunter vor allem das Methyljodid, einzusetzen.

Die Menge des Jods oder der Jodverbindungen beträgt vorzugsweise 0,05-0,2 mol/mol Methanol, und im Falle der anderen, etwas reaktionsträgeren Halogene bzw. von deren Verbindungen etwa bis zum Doppelten dieser Werte.

Methanol und Methyljodid (oder ein anderer definitionsgemäßer Promotor) werden zweckmäßigerweise in flüssiger Form mit dem Kohlenmonoxid vermischt und sodann verdampft, wonach das Gasgemisch in den Reaktor geleitet wird. Nimmt man die Verdampfung erst im Reaktor vor, so empfiehlt es sich dafür zu sorgen, daß der Kontakt möglichst wenig mit der Flüssigkeit in Berührung kommt, etwa indem man den Kontakt in genügendem Abstand vor der Einlaßstelle anordnet.

Da die Carbonylierung exotherm verläuft, muß man für entsprechende Kühlung sorgen. Vorzugsweise verwendet man deshalb einen Rohrbündelreaktor, da dieser eine besonders unproblematische Temperaturführung gestattet.

Die Verweilzeiten der Reaktionspartner betragen etwa 0,02-10 Minuten.

Die Aufarbeitung auf die Verfahrensprodukte kann wie üblich erfolgen, so daß nähere Angaben hierzu entbehrlich sind.

Das erfindungsgemäße Verfahren gestattet Raum-Zeit-Ausbeuten von etwa 0,1-0,3 kg freier oder gebundener Essigsäure pro Stunde und pro Liter Katalysatorschüttung. Auch in rund zweiwöchigem

ununterbrochenem Versuchsbetrieb wurde kein Nachlassen der Katalysatorreaktivität beobachtet, was damit übereinstimmt, daß keine Metallcarbonyle im Reaktoraustrag nachzuweisen waren.

Beispiel 1

Herstellung eines Ni/Pd/Aktivkohle-Trägerkatalysators

200 g Aktivkohle mit der inneren Oberfläche von 600 $m^2/g$, die bei 120 °C und 10 mbar vorbehandelt worden war, wurden mit einer Lösung aus 5 g Palladium-bis(dibenzalaceton) und 1 l Toluol eine Stunde lang bei Raumtemperatur gerührt, danach vom Lösungsmittel abfiltriert und anschließend bei 120 °C und 10 mbar getrocknet.

Diese Masse wurde sodann mit einer Lösung aus 1 l Wasser und 450 g Nickel-II-chlorid-hexahydrat imprägniert, nach Abtrennung der wäßrigen Phase bei 120 °C und 10 mbar getrocknet und 10 min lang mit gasförmigem Ammoniak und danach mit Stickstoff behandelt. Die so erhaltene Katalysator-Vorstufe wurde bei 1 bar und 300 °C zunächst 2 Stunden lang mit einem Gemisch aus 5 Vol.% $H_2$ und 95 Vol.% $N_2$ und dann 8 Stunden lang mit reinem $H_2$ hydrierenden Bedingungen ausgesetzt. Der gebrauchsfertige Trägerkatalysator enthielt 8,7 Gew.% Ni und 0,03 Gew.% Pd (= 0,003 4 Gew.-Teile Pd pro Gew.-Teil Ni).

Beispiel 2

Herstellung eines Ni/Cu/Pd/Aktivkohle-Trägerkatalysators

300 g der Aktivkohle gemäß Beispiel 1 wurden mit einer Lösung aus 10 g Palladiumacetat, 265 g Kupfer-II-chlorid, 259 g Nickel-II-chlorid und 1 100 ml Methanol imprägniert, von der überschüssigen Flüssigkeit abgetrennt, bei 120 °C und 10 mbar getrocknet und 15 min mit gasförmigem Ammoniak und danach mit Argon behandelt.

Nach der hydrierenden Reduktion gemäß Beispiel 1 enthielt der gebrauchsfertigen Trägerkatalysators 3,4 Gew.% Ni, 6,9 Gew.% Cu und 0,5 Gew.% Pd (= 0,15 Gew.-Teile Pd pro Gew.-Teil Ni).

Beispiel 3

Herstellung der Vorstufe eines Ni/Pd/Aktivkohle-Trägerkatalysators

300 g der Aktivkohle gemäß Beispiel 1 wurden mit einer Lösung aus 10 g Palladium-bis(dibenzalaceton) und 1 l Toluol imprägniert, vom überschüssigen Toluol abgetrennt, getrocknet und dieser Behandlung danach auf die gleiche Weise nochmals unterworfen. Anschließend wurde die so erhaltene Masse mit einer Lösung aus 450 g Nickel-II-chlorid und 1 l Wasser imprägniert, abfiltriert und 20 h bei 120 °C und 10 mbar getrocknet. Diese Katalysator-Vorstufe enthielt 6,7 Gew.% Ni und 0,4 Gew.% Pd.

Beispiel 4

Carbonylierung von Methanol mit dem Katalysator gemäß Beispiel 1

Ein Reaktionsrohr von 100 cm Höhe und 1,8 cm lichter Weite wurde mit 137 g (Schüttvolumen 540 g/l) des Katalysators gemäß Beispiel 1 gefüllt und danach bei 300 °C und 1 bar pro Stunde kontinuierlich mit einem Gemisch aus 49,7 g CO, 16,3 g Methanol und 8,4 g Methyljodid (Molverhältnis 30,1 : 8,6 : 1) beschickt.

Der Reaktionsaustrag wurde auf 25 °C gekühlt, wobei eine Gasphase aus CO, 0,8 Vol.% $CH_4$, 0,83 Vol.% Dimethylether und Spuren sonstiger Bestandteile sowie eine Flüssigphase aus Methanol, Methyljodid, 2,0 g Wasser, 5,9 g Essigsäure und 6,1 g Methylacetat anfiel.

Der Methanolumsatz betrug 65 % und die auf das eingesetzte Methanol bezogene Ausbeute an freier und veresterter Essigsäure (Molverhältnis 1,2 : 1) betrug 80 %. Dies entspricht einer Raum-Zeit-Ausbeute von 13 g Essigsäure pro Liter Katalysator pro Stunde.

Die Versuchsdauer betrug 200 Stunden. Innerhalb dieser Zeit war kein Aktivitätsverlust des Katalysators zu beobachten, und es konnten weder Ni noch Pd im Reaktoraustrag nachgewiesen werden.

Beispiel 5

Carbonylierung von Methanol mit dem Katalysator gemäß Beispiel 2

Auf die in Beispiel 4 beschriebene Weise wurden pro Stunde 38,4 g CO, 19,7 g Methanol und 10,3 g Methyljodid (Molverhältnis 18,9 : 8,5 : 1) bei 310 °C an 137 g des Katalysators von Beispiel 2 (Schüttvolumen 540 g/l) umgesetzt.

Die Ausbeute an freier und veresterter Essigsäure (Molverhältnis 2,2 : 1) betrug 73 %, und die Raum-Zeit-Ausbeute betrug 60 g Essigsäure pro Liter Katalysator pro Stunde.

Innerhalb der Versuchsdauer von 120 Stunden war weder ein Aktivitätsverlust des Katalysators zu beobachten, noch konnten Metalle im Reaktoraustrag nachgewiesen werden.

Beispiel 6

Aktivierung der Katalysator-Vorstufe gemäß Beispiel 3 und anschließende Carbonylierung von Methanol

Ein Rohrreaktor von 1 m Höhe und 6 mm lichter Weite wurde mit 15,4 g der Katalysatorvorstufe gemäß Beispiel 3 gefüllt und 12 h bei 280 °C mit einem $H_2$-Strom von 25 bar beschickt.

Im Anschluß an die Hydrierung wurden pro Stunde 31,2 g CO, 13,5 g Methanol und 1,76 g Methyljodid (Molverhältnis 90 : 34 : 1) bei 280 °C und 1 bar durch den Kontakt geleitet.

Die Ausbeuten an freier und veresterter Essigsäure (Molverhältnis 0,3 : 1), bezogen auf das eingesetzte Methanol, betrug 41,6 %, und die Raum-Zeit-Ausbeute betrug 212 g pro Liter Katalysator pro Stunde.

Während einer Versuchsdauer von 280 Stunden blieb die Katalysatoraktivität voll erhalten, und es gelangte kein Metall in den Reaktoraustrag.

**Patentansprüche**

1. Verfahren zur Herstellung von Essigsäure und Methylacetat durch Gasphasencarbonylierung von Methanol in Gegenwart von Nickel enthaltenden Katalysatoren sowie in Gegenwart von Chlor, Brom oder Jod oder flüchtigen Verbindungen dieser Halogene als Promotoren, dadurch gekennzeichnet, daß man hierzu Katalysatoren verwendet, deren aktive Masse aus mindestens 40 Gew.% Nickel besteht und die pro Gew.-Teil Nickel 0,001-1 Gew.-Teile Palladium enthalten.

2. Verfahren nach Anspruch 1, dadurch gekennzeichnet, daß man die Katalysatoren in Form von Trägerkatalysatoren einsetzt.

3. Verfahren nach Anspruch 2, dadurch gekennzeichnet, daß das Trägermaterial der Katalysatoren Aktivkohle ist.

4. Verfahren nach den Ansprüchen 1 bis 3, dadurch gekennzeichnet, daß man als Promotor Methyljodid verwendet.

5. Trägerkatalysatoren, deren aktive Masse aus mindestens 40 Gew.% Nickel besteht und die pro Gew.-Teil Nickel 0,001-1 Gew.-Teile Palladium enthalten.

6. Trägerkatalysatoren nach Anspruch 5, deren Trägermaterial aus Aktivkohle besteht.

**Claims**

1. A process for the preparation of acetic acid and methyl acetate by gas-phase carbonylation of methanol in the presence of a nickel-containing catalyst and in the presence of chlorine, bromine or iodine or a volatile compound of one of these halogens as promoter, wherein the catalyst used is one whose active material consists of at least 40 % by weight of nickel, and the catalyst contains from 0,001 to 1 part by weight of palladium per part by weight of nickel.

2. A process as claimed in claim 1, wherein the catalyst is used in the form of a supported catalyst.

3. A process as claimed in claim 2, wherein the catalyst carrier is active carbon.

4. A process as claimed in claims 1 to 3, wherein the promoter used is methyl iodide.

5. A supported catalyst whose active material consists of at least 40 % by weight of nickel and which contains from 0.001 to 1 part by weight of palladium per part by weight of nickel.

6. A supported catalyst as claimed in claim 5, whose carrier consists of active carbon.

**Revendications**

1. Procédé de préparation d'acide acétique et d'acétate de méthyle par carbonylation en phase gazeuse de méthanol en présence de catalyseurs contenant du nickel, ainsi qu'en présence de chlore, de brome, d'iode ou de composés volatils de ces halogènes servant de promoteurs, caractérisé en ce qu'on utilise à cet effet des catalyseurs dont la masse active se compose d'au moins 40 % en poids de nickel et qui contiennent, par partie en poids de nickel, 0,001 à 1 partie en poids de palladium.

2. Procédé selon la revendication 1, caractérisé en ce qu'on utilise les catalyseurs sous forme de catalyseurs fixés sur support.

3. Procédé selon la revendication 2, caractérisé en ce que la matière de support du catalyseur est un charbon actif.

4. Procédé selon l'une quelconque des revendications 1 à 3, caractérisé en ce qu'on utilise l'iodure de méthyle comme promoteur.

5. Catalyseurs fixés sur support, dont la masse active se compose d'au moins 40 % en poids de nickel

et qui contiennent, par partie en poids de nickel, 0,001 à 1 partie en poids de palladium.

6. Catalyseurs fixés sur support selon la revendication 5, dont la matière de support est faite de charbon actif.